# EUROPEAN PATENT APPLICATION

(11) **EP 4 353 798 A1**
(43) Date of publication of application: **17.04.2024**
(21) Application number: 22811255.3
(22) Date of filing: 20.05.2022
(51) Int. Cl.: C09K 3/00, A61K 8/895, A61K 8/898, A61Q 1/00, A61Q 1/04, A61Q 1/10

(54) **OIL GELLING AGENT AND COSMETIC**

(30) Priority: 28.05.2021 JP 2021089981
(71) Applicant: SHIN-ETSU CHEMICAL CO., LTD., Tokyo 1000005 (JP)
(72) Inventor: HATA, Ryunosuke, Annaka-shi, Gunma 379-0224 (JP)
(74) Representative: Mewburn Ellis LLP
(86) International application number: PCT/JP2022/020929
(87) International publication number: WO 2022/249985

(57) **Abstract**

An oil gelling agent that is a copolymer comprising, as constituting units:
(a) a hydrophilic (meth)acrylic monomer selected from general formulae (1) and (2) 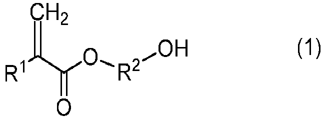 [in formula (1), R¹ represents a hydrogen atom or a methyl group, and R² represents an alkylene group] 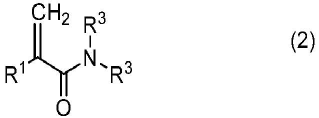 [in formula (2), R¹ represents a hydrogen atom or a methyl group, and R³ represents a group selected from among a hydrogen atom, an alkyl group and a hydroxyalkyl group];
(b) a hydrophobic (meth)acrylic monomer represented by general formula (3) 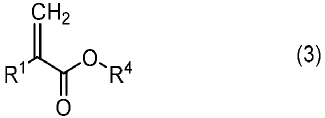 [in formula (3), R¹ represents a hydrogen atom or a methyl group, and R⁴ represents a straight-chain alkyl group having 1-24 carbon atoms]; and
(c) a crosslinker monomer having two or more radical polymerizable functional groups per molecule.

The ratio by mass of (b) to (a) [(b)/(a)] is 0.8-4.0 and the content of monomer (c) in all monomers is 0.05-5 mass%. This oil gelling agent forms a gel having high temperature stability.

## Description

### TECHNICAL FIELD

The present invention relates to an oil gelling agent and to cosmetics containing the oil gelling agent.

### BACKGROUND ART

Oil gelling agents capable of gelling hydrophobic oils and silicone oils are widely used as an ingredient in cosmetics and the like owing to their ability to solidify oils. Hydrocarbon polymers such as polyethylene waxes, long-chain fatty acids and long-chain alcohols are well known as such gelling agents. In addition, compounds which have a sugar backbone as the base and in which some of the hydroxyl groups are acylated are known as compounds of a characteristic structure that are commonly used as oil gelling agents in cosmetics (Patent Document 1). Many gelling agents are thus known, but there remains room for improvement with regard to the temperature stability of the gel that forms.

Gelling agents with an acrylic polymer base are known to be gelling agents that have a relatively high temperature stability (Patent Document 2). By using a hydrophobic monomer and a hydrophilic monomer together and striking a balance in the interactions of both, a gel having a high temperature stability can be formed. However, there remains room for improvement in the temperature stability by way of the type and proportions of the monomers used.

Although gelling agents having an acrylic polymer base are capable of gelling hydrocarbon oils such as isododecane, gelling silicone oils has been difficult.

### PRIOR ART DOCUMENTS

### PATENT DOCUMENTS

Patent Document 1: JP-A 2005-145851
Patent Document 2: WO 2016/098456 A1

### SUMMARY OF INVENTION

### TECHNICAL PROBLEM

In light of the above circumstances, the object of the present invention is to provide an oil gelling agent that forms a gel having high temperature stability.

### SOLUTION TO PROBLEM

The inventor has conducted intensive investigations aimed at achieving this object and has discovered as a result that a copolymer obtained by copolymerizing, in specific proportions, a hydrophilic (meth)acrylic monomer, a hydrophobic (meth)acrylic monomer and a crosslinker monomer having two or more radical polymerizable functional groups on the molecule acts as an oil gelling agent that forms gels having a high temperature stability. This discovery ultimately led to the present invention. Copolymers that have been crosslinked with a crosslinking agent generally have a poor solubility. Also, gels formed with a crosslinked copolymer have a poor feeling on use because they contain three-dimensional crosslinked bodies as microparticles. However, by copolymerizing monomers in the proportions discovered in this invention, a suitable degree of crosslinking occurs, and so it is expected that the above problems can be avoided while increasing the temperature stability.

Accordingly, the invention provides the following oil gelling agent and the following cosmetic containing an oil-gelling agent.
1. An oil gelling agent which is a copolymer containing as constituent units:
   (a) a hydrophilic (meth)acrylic monomer selected from the group consisting of monomers of general formula (1) and monomers of general formula (2) below 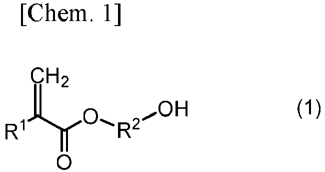 (wherein R¹ is a hydrogen atom or a methyl group, and R² is a linear or branched alkylene group of 2 to 10 carbon atoms) 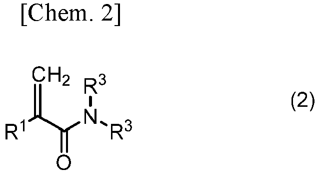 (wherein R¹ is a hydrogen atom or a methyl group; and each R³ is independently a hydrogen atom, a linear or branched alkyl group of 1 to 10 carbon atoms or a hydroxyalkyl group of 1 to 5 carbon atoms),
   (b) a hydrophobic (meth)acrylic monomer of general formula (3) below 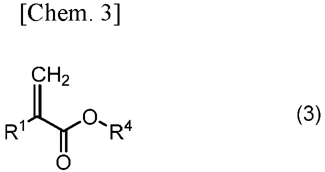 (wherein R¹ is a hydrogen atom or a methyl group, and R⁴ is a linear alkyl group of 1 to 24 carbon atoms), and
   (c) a crosslinker monomer having two or more radical polymerizable functional groups per molecule, wherein the weight ratio (b)/(a) between monomers (a) and (b) is from 0.8 to 4.0 and monomer (c) is included in an amount of from 0.05 to 5 wt% of all the monomers.
2. The oil gelling agent of 1 above, wherein the crosslinker monomer (c) is a di(meth)acrylate-terminated silicone of general formula (4) below 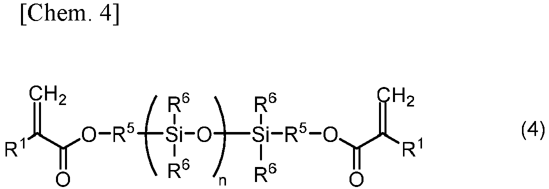 (wherein R¹ is a hydrogen atom or a methyl group; each R⁵ is independently a divalent organic group; each R⁶ is independently a hydrogen atom, a substituted or unsubstituted alkyl group of 1 to 10 carbon atoms, or a substituted or unsubstituted aryl group of 6 to 22 carbon atoms; and n is from 1 to 100).
3. The oil gelling agent of 1 or 2 above, further including:
   (d) a monomer consisting of a silicone macromer of general formula (5) below 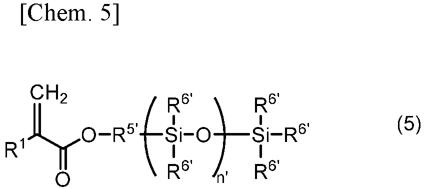 (wherein R¹ is a hydrogen atom or a methyl group; R^{5'} is a divalent organic group; each R^{6'} is independently a hydrogen atom, a substituted or unsubstituted alkyl group of 1 to 10 carbon atoms or a substituted or unsubstituted aryl group of 6 to 22 carbon atoms; and n' is from 1 to 100) as from 10 to 50 wt% of the constituent units in all the monomers.
4. A cosmetic which includes the oil gelling agent of any of 1 to 3 above.

### ADVANTAGEOUS EFFECTS OF INVENTION

A gel having a high temperature stability can be obtained by using the oil gelling agent of this invention.

### DESCRIPTION OF EMBODIMENTS

The invention is described in detail below.

The oil gelling agent of this invention is a copolymer which can be obtained by copolymerizing monomers that include (a) a specific hydrophilic (meth)acrylic monomer, (b) a specific hydrophobic (meth)acrylic monomer and (c) a specific crosslinker monomer having two or more radical polymerizable functional groups per molecule, and which includes the foregoing monomers as the constituent units.

### [(a) Hydrophilic (Meth)Acrylic Monomer]

The hydrophilic (meth)acrylic monomer (a) is a hydrophilic (meth)acrylic monomer selected from compounds of general formula (1) and compounds of general formula (2) below. One such monomer may be used alone or two or more may be used in combination. 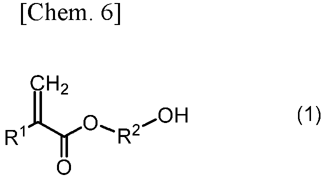 In formula (1), R¹ is a hydrogen atom or a methyl group, and R² is a linear or branched alkylene group of 2 to 10 carbon atoms. 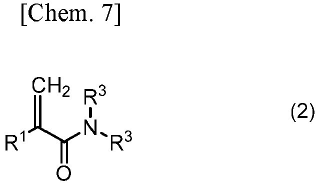 In formula (2), R¹ is a hydrogen atom or a methyl group; and each R³ is independently a hydrogen atom, a linear or branched alkyl group of 1 to 10 carbon atoms or a hydroxyalkyl group of 1 to 5 carbon atoms.

R¹ is a hydrogen atom or a methyl group, and R² is a linear or branched alkylene group of 2 to 10 carbon atoms. From the standpoint of the hydrophilicity, the number of carbon atoms on R² is preferably from 2 to 5, and more preferably 2 or 3.

Each R³ is independently a hydrogen atom, a linear or branched alkyl group of 1 to 10 carbon atoms, or a hydroxyalkyl group of 1 to 5 carbon atoms. From the standpoint of the compatibility with the hydrophobic monomer, a hydrogen atom or an alkyl group of 1 to 3 carbon atoms is preferred; a hydrogen atom or a methyl group is more preferred.

The hydrophilic (meth)acrylic monomers of general formula (1) and general formula (2) may each be used alone or may be used together. From the standpoint of the hydrophilicity, it is preferable to use a hydrophilic (meth)acrylic monomer of general formula (1) alone.

### [(b) Hydrophobic (Meth)acrylic Monomer]

The hydrophobic (meth)acrylic monomer (b) is a hydrophobic (meth)acrylic monomer of general formula (3) below: 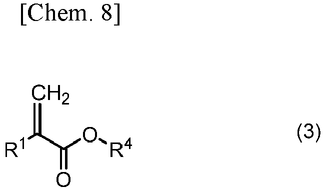 (wherein R¹ is a hydrogen atom or a methyl group, and R⁴ is a linear alkyl group of 1 to 24 carbon atoms).

R⁴ is a linear alkyl group of 1 to 24 carbon atoms. From the standpoint of strengthening hydrophobic interactions, an alkyl group of 8 to 24 carbon groups is preferred; an alkyl group of 16 to 24 carbon atoms is more preferred.

Examples of such hydrophobic (meth)acrylic monomers include, but are not particularly limited to, cetyl acrylate, cetyl methacrylate, stearyl acrylate, stearyl methacrylate, isostearyl acrylate, isostearyl methacrylate, oleyl acrylate, oleyl methacrylate, behenyl acrylate and behenyl methacrylate. One such monomer may be used alone or two or more may be used in combination.

The weight ratio (b)/(a) between monomers (a) and (b) is from 0.8 to 4.0, preferably from 1.0 to 3.8, more preferably from 1.2 to 3.5, and even more preferably from 1.5 to 3.0. At proportions such as these, sufficient hydrophobic interactions arise and, because a wax gel-like gel forms, the feeling on use is also excellent. The combined amount of monomer (a) and monomer (b) is preferably from 95 to 99.95 wt%, more preferably from 97 to 99.93 wt%, and even more preferably from 98.5 to 99.9 wt%, of all the monomers. As used herein, "of all the monomers" means of all the monomers making up the copolymer.

### [(c) Crosslinker Monomer Having Two or More Radical Polymerizable Functional Groups per Molecule]

The crosslinker monomer is not particularly limited so long as it satisfies the above description. For example, glycerol dimethacrylate, polyethylene glycol methacrylate, polypropylene glycol dimethacrylate, polytetramethylene glycol dimethacrylate and ethylene oxide-modified bisphenol A dimethacrylate may be used.

Further examples include di(meth)acrylate-terminated silicones of general formula (4) below 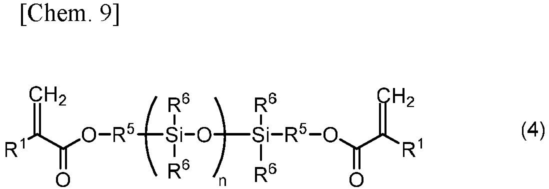 (wherein R¹ is a hydrogen atom or a methyl group; each R⁵ is independently a divalent organic group; each R⁶ is independently a hydrogen atom, a substituted or unsubstituted alkyl group of 1 to 10 carbon atoms, or a substituted or unsubstituted aryl group of 6 to 22 carbon atoms; and n is from 1 to 100). Because the influence exerted by such crosslinking agents on hydrophobic interactions and hydrophilic interactions is small, crosslinking is possible without destroying the balance between hydrophobic monomers and hydrophilic monomers, the crosslinker monomer mixes well with the (meth)acrylate monomers and the reactions proceed without difficulty.

R¹ is a hydrogen atom or a methyl group, each R⁵ is independently a divalent organic group, preferably a divalent hydrocarbon group, more preferably an alkylene group of 2 to 6 carbon atoms, and even more preferably an alkylene group of 2 to 4 carbon atoms. Each R⁶ is independently a hydrogen atom, a substituted or unsubstituted alkyl group of 1 to 10 carbon atoms, or a substituted or unsubstituted aryl group of 6 to 22 carbon atoms. An alkyl group of 1 to 3 carbon atoms is preferred; a methyl group is more preferred. The subscript 'n' is from 1 to 100, preferably from 10 to 90, more preferably from 15 to 80, and even more preferably from 20 to 70.

Examples of the compound of above general formula (4) include, but are not limited, to those shown below.

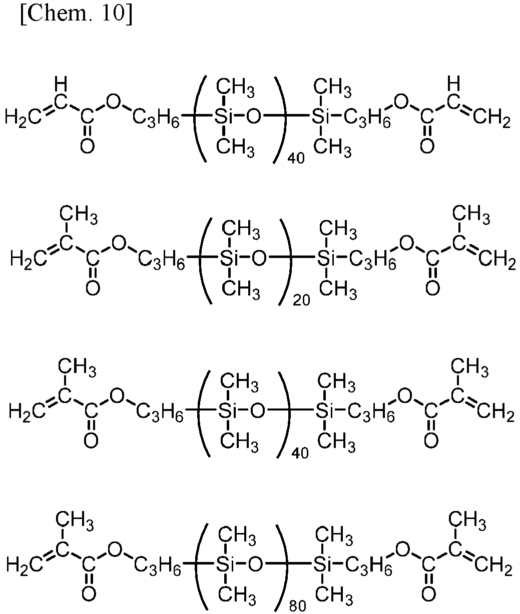

The amount of monomer (c) is from 0.05 to 5 wt%, preferably from 0.07 to 3 wt%, and more preferably from 0.1 to 1.5 wt%, of all the monomers. At this amount, the copolymer crosslinks to a suitable degree, and so the oil gel that forms has a good feeling on use.

### [(d) Silicone Macromer]

The copolymer of the invention, by including as a constituent unit a monomer consisting of a silicone macromer, has an increased compatibility with silicone oils and is better able to gel silicone oils. The silicone macromer is exemplified by silicone macromers of general formula (5) below 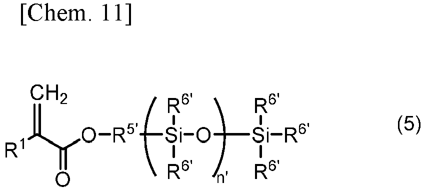 (wherein R¹ is a hydrogen atom or a methyl group; R^{5'} is a divalent organic group; each R^{6'} is independently a hydrogen atom, a substituted or unsubstituted alkyl group of 1 to 10 carbon atoms or a substituted or unsubstituted aryl group of 6 to 22 carbon atoms; and n' is from 1 to 100).

R¹ is a hydrogen atom or a methyl group, R^{5'} is a divalent organic group, and each R^{6'} is independently a hydrogen atom, a substituted or unsubstituted alkyl group of 1 to 10 carbon atoms, or a substituted or unsubstituted aryl group of 6 to 22 carbon atoms. R^{5'} is preferably a divalent hydrocarbon group, more preferably an alkylene group of 2 to 6 carbon atoms, and even more preferably an alkylene group of 2 to 4 carbon atoms.

The compound of general formula (5) is not particularly limited so long as it satisfies the above stipulations. Examples include the compounds shown below 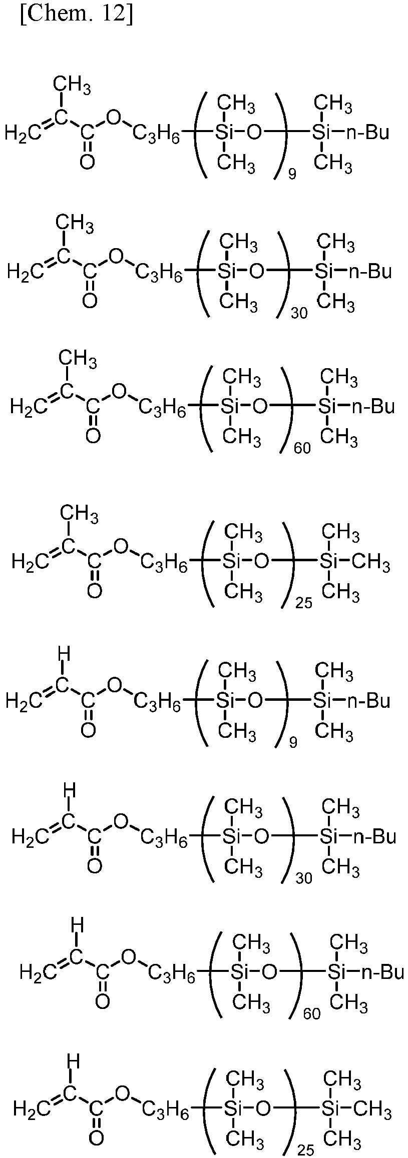 (wherein n-Bu represents the n-butyl group).

In cases where monomer (d) is included as a constituent unit, the amount of monomer (d) is preferably from 10 to 50 wt%, more preferably from 15 to 45 wt%, and even more preferably from 20 to 40 wt%, of all the monomers. At this amount, the function of the copolymer as a gelling agent can be maintained while further increasing its compatibility with silicone oils.

The copolymer of the invention may be composed of above monomers (a) to (c) or monomers (a) to (d) alone, or the copolymer of the invention may additionally include other monomers within ranges that do not compromise the advantageous effects of the invention. When monomers other than the above are included as constituent units, the amount thereof is preferably from 0.05 to 5 wt% of all the monomers. When monomer (d) is used, the combined amount of monomer (a), monomer (b) and monomer (d) is preferably from 95 to 99.95 wt%, more preferably from 97 to 99.93 wt%, and even more preferably from 98.5 to 99.9 wt%, of all the monomers. As used herein, "of all the monomers" means of all the monomers making up the copolymer.

### [Method of Preparing Copolymer]

Polymerization of the copolymer of the invention is preferably carried out in the presence of a radical polymerization initiator such as benzoyl peroxide, lauroyl peroxide or azobisisobutyronitrile. Any of the following polymerization methods may be suitably used: solution polymerization, emulsion polymerization, suspension polymerization, bulk polymerization. Of these, solution polymerization is preferable because adjusting the molecular weight of the resulting polymer within the desired range is easy. The polymerization reaction may be carried out in a solvent. Examples of solvents that may be used at this time include aliphatic hydrocarbon-type organic solvents such as pentane, hexane, decane, dodecane, hexadecane and octadecane; aromatic hydrocarbon-type organic solvents such as benzene, toluene and xylene; alcohol-type organic solvents such as methanol, ethanol, isopropyl alcohol, propanol, butanol, hexanol and decanol; halogenated hydrocarbon-type organic solvents such as chloroform and carbon tetrachloride; and ketone-type organic solvents such as acetone and methyl ethyl ketone. However, from the standpoint of use in cosmetic applications, it is preferable to carry out the polymerization reaction either in the absence of a solvent or to use ethanol or isopropanol. The copolymer may be a random, block or graft copolymer.

The polymer prepared in this way has a polystyrene-equivalent number-average molecular weight (Mn), as determined by GPC, of preferably from 5,000 to 200,000, more preferably from 7,500 to 100,000, and even more preferably from 10,000 to 50,000. Within this molecular weight range, the solubility in oil is even better and the function as a gelling agent can be better maintained. In this invention, "number-average molecular weight" is a value obtained by gel permeation chromatography (GPC) under the following conditions using polystyrene as the reference material.

### [Measurement Conditions]

| | |
|---|---|
| Developing Solvent: | Tetrahydrofuran (THF) |
| Flow rate: | 0.6 mL/min |
| Detector: | Differential refractive index detector (RI) |
| Columns: | TSK Guardcolumn SuperH-H |
| | TSKgel SuperHM-N (one 6.0 mm I.D. × 15 cm column) |
| | TSKgel SuperH2500 (one 6.0 mm I.D. × 15 cm column) |
| Apparatus: (all from Tosoh Corporation) | HLC 8320 GPC |
| Column Temperature: | 40°C |
| Amount of Sample Injected: | 50 µL (THF solution having 0.3 wt% concentration) |

### [Oil Gelling Agent]

The present invention uses the above copolymer as an oil gelling agent. No particular limitation is imposed on the oil that is gelled. Examples include the oils mentioned subsequently as examples of the oil ingredient (B); liquid oil ingredients are preferred. Of these, hydrocarbon oils and silicone oils are preferred.

### [Cosmetic]

The oil gelling agent (A) of the invention can be used in various cosmetic products; use in lip cosmetics is especially suitable. The oil gelling agent forms a gel having a high temperature stability, resulting in a stable composition and improving the feeling on use. The amount of oil gelling agent included in the cosmetic of the invention differs according to the form in which the cosmetic is to be used, although the oil gelling agent may be included in an amount ranging from 0.5 to 99.0 wt% of the overall cosmetic; inclusion in an amount of from 1.0 to 50 wt% of the overall cosmetic is preferred.

Aside from the above oil gelling agent (A), ingredients that are normally used in cosmetics may be included in suitable amounts within the cosmetic of the invention. Such ingredients may include, for example, (B) oil ingredients, (C) ultraviolet-absorbing ingredients, (D) water, (E) surfactants, (F) powders, (G) compounds having an alcoholic hydroxyl group in the molecular structure, (H) water-soluble or water-swellable polymeric compounds, (I) compositions of a crosslinked organopolysiloxane polymer having no hydrophilic groups and a liquid oil, (J) compositions of a crosslinked organopolysiloxane polymer having hydrophilic groups and a liquid oil, (K) silicone resins, and/or (L) silicone waxes. These ingredients are each described below. The compound names given below are sometimes those cited in *Kesho̅hin Hyo̅ji Meisho̅* [Japanese Cosmetic Labeling Names] or the International Nomenclature of Cosmetic Ingredients (INCI). In this Specification, when the name of an ingredient differs in these two lists, the *Kesho̅hin Hyo̅ji Meisho̅* name is given first in lower case letters, followed by, in parenthesis, the capitalized INCI name. When the name from the two lists is the same, it is followed simply by "(INCI)."

### (B) Oil Ingredient

As mentioned above, the cosmetic of the invention may include one or more oil ingredient. Any solid, semisolid or liquid oil that is used in conventional cosmetics may be used as the oil ingredient.

Examples of such liquid oils include one or more silicone oil, hydrocarbon oil, polar oils such as higher fatty acids, ester oils and natural animal and vegetable oils, semisynthetic oils and fluorocarbon oils. Of these, hydrocarbon oils and silicone oils are preferred. Even if the oil ingredient (B) is in this way a silicone oil, because it has compatibility with any oil gelling agent included in the cosmetic of the invention, a cosmetic having a good feeling on use and excellent usability and stability can be provided.

Examples of silicone oils include low-viscosity to high-viscosity linear or branched organopolysiloxanes such as Dimethicone (INCI), Caprylyl Dimethicone (INCI), Phenyl Trimethicone (INCI), Hexyl Dimethicone (INCI), Hydrogen Dimethicone (INCI) and Diphenyl Dimethicone (INCI); cyclic organopolysiloxanes such as Cyclotetrasiloxane (INCI), Cyclopentasiloxane (INCI), Cyclohexasiloxane (INCI), tetrahydrotetramethylcyclotetrasiloxane and tetramethyltetraphenylcyclotetrasiloxane; branched organopolysiloxanes such as tris(trimethylsiloxy)methylsilane and tetrakis(trimethylsiloxy)silane; silicone rubbers such as amino-modified organopolysiloxanes, high degree of polymerization gum-like dimethylpolysiloxanes, gum-like amino-modified organopolysiloxanes and gum-like dimethylsiloxane-methylphenylsiloxane copolymers; cyclic siloxane solutions of a silicone gum or rubber; trimethylsiloxysilicic acid and cyclic organopolysiloxane solutions of trimethylsiloxysilicic acid; higher alkoxy-modified organopolysiloxanes such as Stearoxy Dimethicone (INCI); higher fatty acid-modified organopolysiloxanes; alkyl-modified organopolysiloxanes, linear alkyl-modified organopolysiloxanes, fluorine-modified organopolysiloxanes, silicone resins and dissolved silicone resins.

Hydrocarbon oils are exemplified by linear, branched, and also volatile hydrocarbon oils. Examples include Ozokerite (INCI), olefin oligomers, Isododecane (INCI), Hydrogenated Polyisobutene (INCI), Squalane (INCI), Squalene (INCI), Ceresin (INCI), Paraffin (INCI), Polyethylene (INCI), polyethylene-polypropylene wax, (ethylene/propylene/styrene) copolymer, (butylene/propylene/styrene) copolymer, Pristane (INCI), polyisobutylene, Microcrystalline Wax (INCI) and vaseline (INCI: Petrolatum).

Examples of higher fatty acids include Lauric Acid (INCI), Myristic Acid (INCI), Palmitic Acid (INCI), Stearic Acid (INCI), Behenic Acid (INCI), Undecylenic Acid (INCI), Oleic Acid (INCI), Linoleic Acid (INCI), Linolenic Acid (INCI), Arachidonic Acid (INCI), Eicosapentaenoic Acid (EPA) (INCI), Docosahexaenoic Acid (DHA) (INCI), Isostearic Acid (INCI) and Hydroxystearic Acid (INCI). Examples of higher alcohols include Lauryl Alcohol (INCI), Myristyl Alcohol (INCI), Cetanol (INCI), Stearyl Alcohol (INCI), Behenyl Alcohol (INCI), Oleyl Alcohol (INCI), Isostearyl Alcohol (INCI), Octyldodecanol (INCI), Cetearyl Alcohol (INCI), Decyltetradecanol (INCI), Cholesterol (INCI), Phytosterols (INCI), Batyl Alcohol (INCI) and oleyl glyceryl (INCI: Oleyl Glyceryl Ether).

Examples of ester oils include Diisobutyl Adipate (INCI), Diethylhexyl Adipate (INCI), diphenylundecyl adipate (INCI: Heptylundecyl Adipate), n-alkylene (20-30) monoisostearate glycols, Isocetyl Isostearate (INCI), Trimethylolpropane Triisostearate (INCI), ethylene glycol di-2-ethylhexanoate, cetyl 2-ethylhexanoate (INCI: Cetyl Ethylhexanoate), Trimethylolpropane Triethylhexanoate (INCI), Pentaerythrityl Tetraethylhexanoate (INCI), cetyl octanoate (INCI: Cetyl Ethylhexanoate), Octyldodecyl Myristate (INCI), Oleyl Oleate (INCI), Octyldodecyl Oleate (INCI), Decyl Oleate (INCI), neopentyl glycol dioctanoate (INCI: Neopentyl Glycol Diethylhexanoate), Neopentyl Glycol Dicaprate (INCI), Triethyl Citrate (INCI), Diethylhexyl Succinate (INCI), Amyl Acetate (INCI), Ethyl Acetate (INCI), Butyl Acetate (INCI), Isocetyl Isostearate (INCI), Butyl Stearate (INCI), Diisopropyl Sebacate (INCI), Diethylhexyl Sebacate (INCI), Cetyl Lactate (INCI), Myristyl Lactate (INCI), Isononyl Isononanoate (INCI), Isotridecyl Isononanoate (INCI), Isopropyl Palmitate (INCI), Ethylhexyl Palmitate (INCI), Hexyldecyl Palmitate (INCI), Cholesteryl Hydroxystearate (INCI), Isopropyl Myristate (INCI), Octyldodecyl Myristate (INCI), 2-hexyldecyl myristate, Myristyl Myristate (INCI), hexyldecyl dimethyloctanoate, ethyl laurate (INCI: Ethylhexyl Laurate), Hexyl Laurate (INCI), di(phytosteryl/octyldodecyl) lauroyl glutamate (INCI: Phytosteryl/Octyldodecyl Lauroyl Glutamate), Isopropyl Lauroyl Sarcosinate (INCI) and Diisostearyl Malate (INCI). Examples of glyceride oils include Glyceryl Acetate (INCI), Triethylhexanoin (INCI), glyceryl triisostearate (INCI: Glyceryl Isostearate), Triisopalmitin (INCI), Glyceryl Stearate (INCI), Glyceryl Diisostearate (INCI), Trimyristin (INCI) and glyceryl (isostearate/myristate) (INCI: Isostearic/Myristic Glycerides).

Examples of natural animal and vegetable oils and semisynthetic oils include avocado oil (INCL: Persea Gratissima (Avocado) Oil), linseed oil (INCL: Linum Usitatissimum (Linseed) Seed Oil), almond oil (INCI: Prunus Amygdalus Dulcis (Sweet Almond) Oil), insect wax, perilla oil, olive oil, kapok wax, kaya nut oil, carnauba wax (INCI: Copernicia Cerifera (Carnauba) Wax), Shark Liver Oil (INCI), Cod Liver Oil (INCI), candelilla wax (INCI: Euphorbia Cerifera (Candelilla) Wax), beef tallow, neatsfoot oil, beef bone fat, hydrogenated beef tallow, Prunus Armeniaca (Apricot) Kernel Oil (INCI), spermaceti, Hydrogenated Palm Oil (INCI), wheatgerm oil (INCI: Triticum Vulgare (Wheat) Germ Oil), sesame oil (INCI: Sesamum Indicum (Sesame) Seed Oil), rice germ oil (INCI: Oryza Sativa (Rice) Germ Oil), rice bran oil (INCI: Oryza Sativa (Rice) Bran Oil), sugarcane wax (INCI: Saccharum Officinarum (Sugarcane) Wax), Camellia Kissi Seed Oil (INCI), safflower oil (INCI: Carthamus Tinctorius (Safflower) Seed Oil), shea butter (INCI: Butyrospermum Parkii (Shea Butter)), coconut oil (INCI: Cocos Nucifera (Coconut) Oil), cinnamon oil, jojoba wax (INCI: Simmondsia Chinensis (Jojoba) Seed Wax), Shellac Wax (INCI), Turtle Oil (INCI), soybean oil (INCI: Glycine Soja (Soybean) Oil), Camellia Sinensis Seed Oil (INCI), Camellia Japonica Seed Oil (INCI), evening primrose oil (Oenothera Biennis (Evening Primrose) Oil, corn oil (INCI: Zea Mays (Corn) Oil), Lard (INCI), rapeseed oil, tung oil, rice bran wax (INCI: Oryza Sativa (Rice) Bran Wax), Horse Fat (INCI), persic oil, palm oil (INCI: Elaeis Guineensis (Palm) Oil), palm kernel oil (INCI: Elaeis Guineensis (Palm) Kernel Oil), castor oil (INCI: Ricinus Communis (Castor) Seed Oil), Hydrogenated Castor Oil (INCI), castor oil fatty acid methyl ester, sunflower oil, grapeseed oil (INCI: Vitis Vinifera (Grape) Seed Oil), bayberry wax, Jojoba Oil (INCI), Hydrogenated Jojoba Oil (INCI), macadamia nut oil (INCI: Macadamia Integrifolia Seed Oil), Beeswax (INCI), Mink Oil (INCI), meadowfoam oil (INCI: Limnanthes Alba (Meadowfoam) Seed Oil), cottonseed oil (INCI: Gossypium (Cotton) Seed Oil), cotton wax, Japan wax (INCI: Rhus Succedanea Fruit Wax), Montan Wax (INCI), Hydrogenated Coconut Wax (INCI), glyceryl tricocoate, peanut oil (INCI: Arachis Hypogaea (Peanut) Oil), Lanolin (INCI), liquid lanolin (INCI: Lanolin Oil), Hydrogenated Lanolin (INCI), Lanolin Alcohol (INCI), Lanolin Wax (INCI), Acetylated Lanolin (INCI), Acetylated Lanolin Alcohol (INCI), Isopropyl Lanolate (INCI), POE lanolin alcohol ethers, PEO lanolin alcohol acetates, polyethylene glycol lanolate, PEO hydrogenated lanolin alcohol ethers and egg yoke oil (INCI: Egg Oil). Here, "POE" stands for polyoxyethylene.

Fluorocarbon oils include perfluoropolyethers, Perfluorodecalin (INCI) and perfluorooctane.

The amount of oil ingredient (B) included in the cosmetic of the invention differs depending on the form in which the cosmetic is to be used, but is preferably from 1 to 98 wt%, and more preferably from 1 to 50 wt%, of the overall cosmetic.

### (C) Ultraviolet-Absorbing Ingredient

The cosmetic of the invention may also include one or more ultraviolet-absorbing ingredient. By doing so, the inventive cosmetic, in addition to having a good feeling on use and excellent usability and staying power, becomes a cosmetic which can absorb ultraviolet rays. Ultraviolet-absorbing ingredients encompass ultraviolet absorbers and ultraviolet-scattering agents. Examples of ultraviolet absorbers include benzoic acid-type ultraviolet absorbers such as PABA (INCI), anthranilic acid-type ultraviolet absorbers such as Methyl Anthranilate (INCI), salicylic acid-type ultraviolet absorbers such as Methyl Salicylate (INCI), cinnamic acid-type ultraviolet absorbers such as octyl methoxycinnamate (INCI: Ethylhexyl Methoxycinnamate), benzophenone-type ultraviolet absorbers such as oxybenzone-1 (INCI: Benzophenone-1), urocanic acid-type ultraviolet absorbers such as ethyl urocanate, and dibenzoylmethane-type ultraviolet absorbers such as t-butyl methoxydibenzoylmethane (INCI: Butyl Methoxydibenzoymethane). The earlier described silicone derivatives having functional groups with ultraviolet-absorbing properties may also be used. Ultraviolet absorbing and scattering agents are exemplified by powders that absorb and scatter ultraviolet light, including microparticulate titanium oxide, microparticulate iron-containing titanium oxide, microparticulate zinc oxide, microparticulate cerium oxide, and composites thereof. Of these, cinnamic acid-type ultraviolet absorbers, dibenzoylmethane-type ultraviolet absorbers, titanium oxide and zinc oxide are preferred.

### (D) Water

Water may be included in the cosmetic of the invention, depending on the purpose of the cosmetic. The cosmetic of the invention, by including water according to the purpose of use thereof, becomes a cosmetic having an even better usability. The amount of water included is preferably not more than 95 wt% of the overall cosmetic.

### (E) Surfactant

The cosmetic of the invention may also include one or more surfactant. The cosmetic of the invention, by including a surfactant according to the purpose of use thereof, becomes a cosmetic having an even better usability. There are anionic, cationic, nonionic and amphoteric surfactants, although the type of surfactant included in the cosmetic of the invention is not particularly limited. Use can be made of any surfactant employed in conventional cosmetics.

Examples of anionic surfactants include fatty acid soaps such as Sodium Stearate (INCI) and TEA-Palmitate (INCI); alkyl ether carboxylic acids and salts thereof; amino acid-fatty acid condensates; alkanesulfonates, alkenesulfonates, sulfonates of fatty acid esters, sulfonates of fatty acid amides and formalin-condensed sulfonates; sulfate salts such as alkyl sulfates, secondary higher alcohol sulfates, alkyl and aryl ether sulfates, sulfates of fatty acid esters, sulfates of fatty acid alkylolamides and Sulfated Castor Oil (INCI); alkyl phosphates, ether phosphates, alkyl aryl ether phosphates, amide phosphates, N-acyl lactates, N-acyl sarcosinates and N-acylamino acid-type surfactants. Examples of cationic surfactants include amine salts such as alkylamine salts and polyamine and aminoalcohol fatty acid derivatives, alkyl quaternary ammonium salts, aromatic quaternary ammonium salts, pyridinium salts and imidazolium salts.

Examples of nonionic surfactants include sorbitan fatty acid esters, glycerin fatty acid esters, polyglycerin fatty acid esters, propylene glycol fatty acid esters, polyethylene glycol fatty acid esters, sucrose fatty acid esters, methylglucoside fatty acid esters, alkyl polyglucosides, polyoxyethylene alkyl ethers, polyoxypropylene alkyl ethers, polyoxyethylene alkyl phenyl ethers, polyoxyethylene fatty acid esters, polyoxyethylene sorbitan fatty acid esters, polyoxyethylene sorbitol fatty acid esters, polyoxyethylene glycerin fatty acid esters, polyoxyethylene propylene glycol fatty acid esters, polyoxyethylene castor oil, polyoxyethylene hydrogenated castor oil, polyoxyethylene phytostanol ether, polyoxyethylene phytosterol ether, polyoxyethylene cholestanol ether, polyoxyethylene cholesterol ether, linear or branched polyoxyalkylene-modified organopolysiloxanes, linear or branched polyoxyalkylene/alkyl co-modified organopolysiloxanes, linear or branched polyglycerin-modified organopolysiloxanes, linear or branched polyglycerin/alkyl co-modified organopolysiloxanes, alkanolamides, sugar ethers and sugar amides.

Examples of amphoteric surfactants include betaine, aminocarboxylic acid salts, imidazoline derivatives and amidoamine compounds.

Of these surfactants, linear or branched organopolysiloxanes having polyoxyethylene chains on the molecule, linear or branched organopolysiloxanes having polyglycerin chains on the molecule, and surfactants which are alkyl co-modified organopolysiloxanes of each are preferred. Examples of commercial products include, without particular limitation, KF-6011, KF-6011P, KF-6043, KF-6012, KF-6013, KF-6015, KF-6016, KF-6017, KF-6028, KF-6028P, KF-6038, KF-6100, KF-6104, KF-6105 and KF-6106 (all from Shin-Etsu Chemical Co., Ltd.). Surfactants having a hydrophilic-lipophilic balance (HLB) of from 2 to 10 are preferred. The amount of surfactant included is preferably from 0.1 to 20 wt%, and more preferably from 0.2 to 10 wt%, of the overall cosmetic.

### (F) Powder

The cosmetic of the invention may also include one or more powder. So long as the powder is one that is used in conventional cosmetics, use can be made of a powder of any shape (spherical, needle-like, platy, etc.), particle size (aerosol-like, fine particles, pigment grade, etc.) and particle structure (porous, nonporous, etc.). Exemplary powders include inorganic powders, organic powders, surfactant metal salt powders, and colorants such as color pigments, pearlescent pigments, coal tar dyes, metal powder pigments, natural colors, and dyes.

Examples of inorganic powders include Titanium Dioxide (INCI), zirconium oxide (INCI: Zirconium Dioxide), Zinc Oxide (INCI), Cerium Oxide (INCI), magnesium oxide, barium sulfate, calcium sulfate, magnesium sulfate, calcium carbonate, magnesium carbonate, Talc (INCI), Mica (INCI), Kaolin (INCI), sericite, synthetic mica, phlogopite, red lepidolite, biotite, lepidolite, Silica (INCI), Aluminum Silicate (INCI), Magnesium Silicate (INCI), Magnesium Aluminum Silicate (INCI), Calcium Silicate (INCI), barium silicate, strontium silicate, metal salts of tungstic acid, Hydroxyapatite (INCI), vermiculite, hydrite, Bentonite (INCI), Montmorillonite (INCI), Hectorite (INCI), Zeolite (INCI), dibasic calcium phosphate, Alumina (INCI), Aluminum Hydroxide (INCI), Boron Nitride (INCI) and Silica Silylate (INCI).

Examples of organic powders include polyamide powder, polyacrylic acid/polyacrylate ester powder, polyester powder, polyethylene powder, polypropylene powder, polystyrene powder, polyurethane, benzoguanamine powder, polymethyl benzoguanamine powder, tetrafluoroethylene powder, polymethyl methacrylate powder, Cellulose (INCI), silk powder, nylon powder, Nylon-12 (INCI), Nylon-6 (INCI), crosslinked spherical dimethylpolysiloxane fine powders having a structure in which the dimethylpolysiloxane is crosslinked, crosslinked spherical polymethylsilsesquioxane fine powder, a fine powder in which crosslinked spherical organopolysiloxane rubber surfaces are covered with polymethylsilsesquioxane particles, hydrophobized silica, styrene-acrylic acid copolymers, divinylbenzene-styrene copolymers, vinyl resins, urea resins, phenolic resins, fluorocarbon resins, silicone resins, acrylic resins, melamine resins, epoxy resins, polycarbonate resins, microcrystalline fiber powder, starch powder, fatty acid starch derivative powder and Lauroyl Lysine (INCI).

Examples of surfactant metal salt powders (metal soaps) include Zinc Undecylenate (INCI), Aluminum Isostearate (INCI), Zinc Stearate (INCI), Aluminum Stearate (INCI), Calcium Stearate (INCI), Magnesium Stearate (INCI), Zinc Myristate (INCI), Magnesium Myristate (INCI), Sodium Zinc Cetyl Phosphate (INCI), calcium cetyl phosphate, Zinc Palmitate (INCI), aluminum palmitate and Zinc Laurate (INCI).

Specific examples of color pigments include inorganic red pigments such as iron oxide, iron hydroxide and iron titanate, inorganic brown pigments such as γ-iron oxide, inorganic yellow pigments such as yellow iron oxide and yellow ocher, inorganic black pigments such as black iron oxide and Carbon Black (INCI), inorganic violet pigments such as Manganese Violet (INCI) and cobalt violet, inorganic green pigments such as chromium hydroxide (INCI: Chromium Hydroxide Green), chromium oxide (INCI: Chromium Oxide Greens), cobalt oxide and cobalt titanate (INCI: Cobalt Titanium Oxide), inorganic blue pigments such as Prussian blue and ultramarine blue, lakes of coal tar dyes, lakes of natural dyes, and synthetic resin powders that are composites of these powders.

Specific examples of pearlescent pigments include titanium oxide-coated mica, bismuth oxychloride, titanium oxide-coated bismuth oxychloride, titanium oxide-coated talc, natural pearl essence, and titanium oxide-coated colored mica. Examples of metal powder pigments include aluminum powder, copper powder and stainless steel powder.

Examples of coal tar dyes include red No. 3, red No. 104, red No. 106, red No. 201, red No. 202, red No. 204, red No. 205, red No. 220, red No. 226, red No. 227, red No. 228, red No. 230, red No. 401, red No. 505, yellow No. 4, yellow No. 5, yellow No. 202, yellow No. 203, yellow No. 204, yellow No. 401, blue No. 1, blue No. 2, blue No. 201, blue No. 404, green No. 3, green No. 201, green No. 204, green No. 205, orange No. 201, orange No. 203, orange No. 204, orange No. 206 and orange No. 207. Examples of natural colors include powders selected from carminic acid, Laccaic Acid (INCI), carthamin, brazilin and crocin.

Of these powders, in the present invention, crosslinked spherical dimethylpolysiloxane fine powders having a structure crosslinked with dimethylpolysiloxane, crosslinked spherical polymethylsilsesquioxane fine powder, a fine powder in which crosslinked spherical polysiloxane rubber surfaces are coated with polymethylsilsesquioxane particles, a fine powder in which crosslinked spherical diphenyl polysiloxane rubber surfaces are covered with polymethylsilsesquioxane particles, and hydrophobized silica are preferred. Powders and colorants having fluorine groups may also be used. Commercial products include KMP-590, KSP-100, KSP-101, KSP-102, KSP-105 and KSP-300 (all from Shin-Etsu Chemical Co., Ltd.).

Composites of these powders and products obtained by treating these powders with a common oil, silicone oil, fluorocarbon compound, surfactant or the like may also be used, insofar as doing so does not detract from the advantageous effects of the invention. The powder may or may not be surface-treated beforehand by way of, for example, fluorine compound treatment, silicone resin treatment, "pendant" treatment, silane coupling agent treatment, titanium coupling agent treatment, oil treatment, N-acylated lysine treatment, polyacrylic acid treatment, metal soap treatment, amino acid treatment, inorganic compound treatment, plasma treatment and mechanochemical treatment. If necessary, one, two or more such treatments may be used. It is preferable to include these powders in an amount within a range of up to 99 wt% of the overall cosmetic. In the case of powder cosmetics in particular, the amount included is preferably in the range of 80 to 99 wt% of the overall cosmetic.

### (G) Compounds Having Alcoholic Hydroxyl Group in Molecular Structure

The cosmetic of the invention may also include one or more compound having an alcoholic hydroxyl group in the molecular structure. Examples of such compounds include lower alcohols such as ethanol (INCI: Alcohol) and Isopropyl Alcohol (INCI); sugar alcohols such as Sorbitol (INCI) and Maltose (INCI); sterols such as Cholesterol (INCI), Beta-Sitosterol (INCI), Phytosterols (INCI) and Lanosterol (INCI); and polyhydric alcohols such as BG (INCI: Butylene Glycol), PG (INCI: Propylene Glycol), DPG (INCI: Dipropylene Glycol) and Pentylene Glycol (INCI). In general, water-soluble monohydric alcohols and water-soluble polyhydric alcohols are often used. Compounds having an alcoholic hydroxyl group in the molecular structure are included in an amount which is preferably not more than 98 wt% of the overall cosmetic.

### (H) Water-Soluble or Water-Swellable Polymeric Compound

The cosmetic of the invention may also include one or more water-soluble or water-swellable polymeric compound. Examples of these water-soluble or water-swellable polymeric compounds include plant-derived polymeric compounds such as gum arabic (INCI: Acacia Senegal Gum), tragacanth gum tree gum (INCI: Astragalus Gummifer Gum), galactan, locust bean gum (INCI: Ceratonia Siliqua Gum), guar gum (INCI: Cyamopsis Tetragonoloba (Guar) Gum), karaya gum (INCI: Sterculia Urens Gum), carrageenan (INCI: Chondrus Crispus (Carrageenan)), Pectin (INCI), Agar (INCI), quince seed (INCI: Pyrus Cydonia Seed), rice starch (INCI: Oryza Sativa (Rice) Starch), wheat starch (INCI: Triticum Vulgare (Wheat) Starch), potato starch (INCI: Solanum Tuberosum (Potato) Starch) and tragacanth (INCI: Astragalus Gummifer Gum); microorganism-derived polymeric compounds such as Xanthan Gum (INCI), Dextran (INCI), Succinoglycan (INCI) and Pullulan (INCI); animal-derived polymeric compounds such as Collagen (INCI), Casein (INCI), albumin and Gelatin (INCI); starch-based polymeric compounds such as Sodium Carboxymethyl Starch (INCI) and Hydroxypropyl Starch (INCI); cellulosic polymer compounds such as Methylcellulose (INCI), Ethylcellulose (INCI), Hydroxypropyl Methylcellulose (INCI), carboxymethylcellulose, hydroxymethylcellulose, Hydroxypropylcellulose (INCI), Nitrocellulose (INCI), Sodium Cellulose Sulfate (INCI), sodium carboxymethylcellulose, Microcrystalline Cellulose (INCI) and cellulose powder; alginic acid-based polymeric compounds such as sodium alginate (INCI: Algin), PG alginate (INCI: Propylene Glycol Alginate); vinyl-based polymeric compounds such as polyvinyl methyl ether and carboxyvinyl polymer; polyoxyethylene-based polymeric compounds; polyoxyethylene-polyoxypropylene copolymer-based polymeric compounds; acrylic polymers such as Sodium Polyacrylate (INCI), Polyethylacrylate (INCI), Polyacrylamide (INCI) and Acrylamide/Sodium Acryloyldimethyltaurate Copolymer (INCI); other synthetic water-soluble polymeric compounds such as polyethyleneimine and cationic polymers; and inorganic water-soluble polymer compounds such as Bentonite (INCI), Magnesium Aluminum Silicate (INCI), Montmorillonite (INCI), beidellite, nontronite, saponite, Hectorite (INCI) and Silica (INCI). Film-forming agents such as Polyvinyl Alcohol (INCI) and PVP (INCI) are also included among these water-soluble polymeric compounds. The water-soluble or water-swellable polymeric compound is included in an amount of preferably not more than 25 wt% of the overall cosmetic.

### (I) Composition of Hydrophilic Group-Free Crosslinked Organopolysiloxane Polymer and Liquid Oil

The cosmetic of the invention may also include (I) one or more composition consisting of a hydrophilic group-free crosslinked organopolysiloxane polymer and a liquid oil. The crosslinked organopolysiloxane polymer can be obtained by reacting an alkyl hydrogen polysiloxane with a hydrosilylating, reactive unsaturated group-containing crosslinking agent. Examples of the alkyl hydrogen polysiloxane include methyl hydrogen polysiloxanes having linear or partially branched units, and methyl hydrogen polysiloxanes to which have been grafted alkyl chains of from 6 to 20 carbon atoms. The molecule must have an average of two or more silicon-bonded hydrogen atoms thereon. Examples of the crosslinking agent include compounds having on the molecule two or more carbon-carbon double bonds capable of a hydrosilylation reaction, such as methyl vinyl polysiloxane and α,ω-alkenyldienes. The resulting crosslinked organopolysiloxane polymer is made to swell with its own weight or more of a low-viscosity silicone having a kinematic viscosity at 25°C of from 0.65 to 100.0 mm²/s, a hydrocarbon oil such as liquid paraffin, squalane or isododecane, a glyceride oil such as trioctanoin, or a liquid oil such as an ester oil.

Compositions of these crosslinked organopolysiloxane polymers and liquid oils are available as commercial products. Examples include KSG-15, KSG-16, KSG-18, KSG-1610 and USG-103, which have been rendered into the form of a paste with a silicone oil; and USG-106, KSG-41, KSG-42, KSG-43, KSG-44 and KSG-810, which have been rendered into the form of a paste with a hydrocarbon oil or a triglyceride oil. These are all products of Shin-Etsu Chemical Co., Ltd. The composition (I) consisting of a hydrophilic group-free crosslinked organopolysiloxane and a liquid oil is included in an amount of preferably from 0.1 to 50 wt%, and more preferably from 1 to 30 wt%, of the overall cosmetic.

### (J) Composition of Hydrophilic Group-Containing Crosslinked Organopolysiloxane Polymer and Liquid Oil

The cosmetic of the invention may also include one or more composition consisting of a hydrophilic group-containing crosslinked organopolysiloxane polymer and a liquid oil. The hydrophilic group is preferably a polyether group or a polyglyceryl group. A polyether group and/or polyglycerol group-containing crosslinked organopolysiloxane polymer can be obtained by reacting an alkyl hydrogen polysiloxane with a hydrosilylating, reactive unsaturated group-containing crosslinking agent. Examples of the alkyl hydrogen polysiloxane include methyl hydrogen polysiloxanes to which have been grafted polyoxyethylene chains and methyl hydrogen polysiloxanes to which have been grafted polyglycerol chains. The molecule must have an average of two or more silicon-bonded hydrogen atoms thereon.

Examples of the crosslinking agent include compounds having on the molecule two or more carbon-carbon double bonds capable of a hydrosilylating reaction, such as α,ω-alkenyldienes, glycerol triallyl ether, polyoxyalkynylated glycerol triallyl ether, trimethylolpropane triallyl ether and polyoxyalkynylated trimethylolpropane trially ether. The crosslinked product obtained by reacting these is a compound having at least one hydrophilic group.

The resulting crosslinked organopolysiloxane polymer is made to swell with its own weight or more of a low-viscosity silicone having a kinematic viscosity at 25°C of from 0.65 to 100.0 mm²/s, a hydrocarbon oil such as liquid paraffin, squalane or isododecane, a glyceride oil such as trioctanoin, or a liquid oil such as an ester oil.

These crosslinked organopolysiloxanes are available as commercial products. Examples include KSG-210, KSG-240, KSG-710, which have been rendered into the form of a paste with a silicone oil; and KSG-310, KSG-320, KSG-330, KSG-340, KSG-820, KSG-830 and KSG-840, which have been rendered into the form of a paste with a hydrocarbon oil or a triglyceride oil. These are all products of Shin-Etsu Chemical Co., Ltd. The composition (J) consisting of a hydrophilic group-containing crosslinked organopolysiloxane and a liquid oil is included in an amount of preferably from 0.1 to 50 wt%, and more preferably from 1 to 30 wt%, of the overall cosmetic.

### (K) Silicone Resin

The cosmetic of the invention may also include (K) one or more silicone resin. The silicone resin is preferably one selected from the group consisting of silicone network compounds containing SiO_{4/2} and/or R'SiO_{3/2} (wherein R' is an alkyl group) units, linear acrylic/silicone grafts, and block copolymers thereof. The linear acrylic/silicone graft or block copolymer may have one or more moiety selected from pyrrolidone moieties, linear alkyl moieties, polyoxyalkylene moieties, fluoroalkyl moieties, and anionic moieties of carboxylic acids and the like. Examples of commercial products include, without particular limitation, the following dissolved in a silicone oil, hydrocarbon oil or alcohol: KP-541, KP-543, KP-545, KP-549, KP-550, KP-571, KP-575 and KP-581 (all from Shin-Etsu Chemical Co., Ltd.).

The silicone network compound is preferably a silicone network compound denoted as MQ, MDQ, MT, MDT or MDTQ. Here, M, D, T and Q respectively stand for R₃SiO_{1/2} units, R₂SiO_{2/2} units, RSiO_{3/2} units and SiO_{4/2} units. The silicone network compound may include on the molecule one or more moiety selected from pyrrolidone moieties, linear alkyl moieties, polyoxyalkylene moieties, fluoroalkyl moieties and amino moieties. Examples of commercial products include, without particular limitation: KF-7312J, KF-7312K and KF-7321T (all from Shin-Etsu Chemical Co., Ltd.).

The silicone resin (K) may be one that is dissolved in a low-viscosity silicone oil, a volatile silicone oil or another solvent. The amount of silicone resin included in each case is such that the resin accounts for preferably from 0.1 to 20 wt%, and more preferably from 1 to 10 wt%, of the overall cosmetic of the invention.

### (L) Silicone Wax

The cosmetic of the invention may also include, depending on the intended purpose thereof, (L) a silicone wax. This silicone wax is exemplified by polylactone-modified polysiloxanes in which is bonded a polylactone that is a ring-opening polymer of a lactone compound having at least a 5-membered ring, and acrylic-modified polysiloxanes containing on the molecule one or more functional group selected from pyrrolidone groups, linear alkyl groups, polyoxyalkylene groups, fluoroalkyl groups and anionic groups of carboxylic acids and the like. Examples of commercial products that are linear alkyl group-containing waxes include KP-561P and KP-562P (both from Shin-Etsu Chemical Co., Ltd.).

Examples of silicone waxes include silicone-modified olefin waxes obtained by addition reacting an olefin wax with an organohydrogen polysiloxane having one or more SiH bond on the molecule. The olefin wax is one obtained by copolymerizing ethylene and one or more diene, or one obtained by copolymerizing one or more olefin selected from among ethylene and α-olefins having 3 to 12 carbon atoms with one or more diene. This diene is preferably vinyl norbornene.

When a silicone wax is used, the amount included is preferably from 0.1 to 20 wt%, and more preferably from 1 to 10 wt%, of the overall cosmetic.

### Other Ingredients

Ingredients used in conventional cosmetics may also be added to the cosmetic of the invention within ranges that do not detract from the advantageous effects of the invention. Examples of such ingredients include oil-soluble gelling agents, antiperspirants, moisturizers, antimicrobial preservatives, salts, antioxidants, skin beautifying ingredients (whitening agents, cell activators, skin roughness improvers, circulation promoting ingredients, skin astringents, antiseborrheic agents, etc.), vitamins, amino acids and hair-fixing polymer compounds.

Examples of oil-soluble gelling agents include one or more selected from metal soaps such as Aluminum Stearate (INCI), Magnesium Stearate (INCI) and Zinc Myristate (INCI); amino acid derivatives such as Lauroyl Glutamic Acid (INCI); dextrin fatty acid esters such as Dextrin Palmitate (INCI), Dextrin Isostearate (INCI) and Dextrin Palmitate/Ethylhexanoate (INCI); sucrose fatty acid esters such as Sucrose Palmitate (INCI) and Sucrose Stearate (INCI); fructooligosaccharide fatty acid esters such as Fructooligosaccharide Caprylate (INCI) and Fructooligosaccharide Hexyldecanoate (INCI); benzylidene derivatives of sorbitol, such as monobenzylidene sorbitol and dibenzylidene sorbitol; and organic-modified clay minerals such as dimethylbenzyl dodecylammonium montmorillonite and dimethyldioctadecylammonium montmorillonite.

Examples of antiperspirants include one or more selected from Aluminum Chlorohydrate (INCI), Aluminum Chloride (INCI), aluminum sesquichlorohydrate, zirconium hydroxychloride, aluminum-zirconium hydroxychloride and aluminum-zirconium glycine complexes.

Examples of moisturizers include one or more selected from Glycerin (INCI), Sorbitol (INCI), PG (INCI), DPG (INCI), BG (INCI), Pentylene Glycol (INCI), Glucose (INCI), Xylitol (INCI), Maltitol (INCI), polyethylene glycol, Hyaluronic Acid (INCI), chondroitin sulfate, pyrrolidonecarboxylic acid salts, polyoxyethylene methyl glucoside and polyoxypropylene methyl glucoside.

Examples of antimicrobial preservatives include one or more selected from alkyl p-hydroxybenzoates, Benzoic Acid (INCI), Sodium Benzoate (INCI), Sorbic Acid (INCI), Potassium Sorbate (INCI) and Phenoxyethanol (INCI). Examples of antimicrobial agents include benzoic acid, salicylic acid, Phenol (INCI), sorbic acid, alkyl p-hydroxybenzoates, p-Chloro-m-Cresol (INCI), Hexachlorophene (INCI), Benzalkonium Chloride (INCI), Chlorhexidine Dihydrochloride (INCI), Triclocarban (INCI), photosensitizers and phenoxyethanol.

Exemplary salts include inorganic salts, organic acid salts, amine salts and amino acid salts. One, two or more salts may be selected from among inorganic salts such as the sodium, potassium, magnesium, calcium, aluminum, zirconium and zinc salts of inorganic acids such as hydrochloric acid, sulfuric acid, carbonic acid and nitric acid; salts of organic acids such as Acetic Acid (INCI), Dehydroacetic Acid (INCI), Citric Acid (INCI), Malic Acid (INCI), Succinic Acid (INCI), Ascorbic Acid (INCI) and Stearic Acid (INCI); and salts of amines such as TEA (INCI: Triethanolamine) and salts of amino acids such as Glutamic Acid (INCI). In addition, use can be made of salts of Hyaluronic Acid (INCI), chondroitin sulfuric acid and the like, Aluminum Zirconium Tetrachlorohydrate (INCI), and also acid-alkali neutralization salts used in cosmetic formations.

Examples of antioxidants include Tocopherol (INCI), BHA (INCI), BHT (INCI) and Phytic Acid (INCI). Examples of pH adjustors include Lactic Acid (INCI), Citric Acid (INCI), Glycolic Acid (INCI), Succinic Acid (INCI), Tartaric Acid (INCI), Malic Acid (INCI), Potassium Carbonate (INCI), Sodium Bicarbonate (INCI) and Ammonium Bicarbonate (INCI). Examples of chelating agents include Alanine (INCI), Disodium EDTA (INCI), Sodium Polyphosphate (INCI), Sodium Metaphosphate (INCI) and Phosphoric Acid (INCI). Examples of algefacients include Menthol (INCI) and camphor. Examples of anti-inflammatory agents include Allantoin (INCI), Glycyrrhizic Acid (INCI) and salts thereof, Glycyrrhetinic Acid (INCI), Stearyl Glycyrrhetinate (INCI), tranexamic acid and Azulene (INCI).

Examples of skin beautifying agents include one, two or more selected from whitening agents such as Placental Extract (INCI), Albutin (INCI), Glutathione (INCI) and Saxifraga Sarmentosa Extract (INCI); cell activators such as Royal Jelly (INCI), photosensitizers, cholesterol derivatives and calf blood extract; skin roughness improvers; circulation promoting agents such as nonanoic acid vanillylamide, Benzyl Nicotinate (INCI), β-butoxyethyl nicotinate, Capsaicin (INCI), Acetyl Zingerone (INCI), cantharides tincture, Ichthammol (INCI), Caffeine (INCI), tannic acid, Borneol (INCI), Tocopheryl Nicotinate (INCI), inositol hexanicotinate, cyclandelate, cinnarizine, tolazoline, acetylcholine, verapamil, cepharanthine and Oryzanol (INCI); skin astringents such as Zinc Oxide (INCI) and Tannic Acid (INCI); and anti seborrheic agents such as Sulfur (INCI) and thianthrol.

Examples of vitamins include one, two or more selected from vitamin A oil and vitamin A compounds such as Retinol (INCI), Retinyl Acetate (INCI) and Retinyl Palmitate (INCI); vitamin B compounds, including the vitamin B2 compounds Riboflavin (INCI), Riboflavin Tetrabutyrate (INCI) and Disodium Flavine Adenine Dinucleotide (INCI), the vitamin B6 compounds Pyrodoxine HCl (INCI), pyridoxine dioctanoate and Pyridoxine Dipalmitate (INCI), vitamin B 12 and derivatives thereof, and vitamin B 15 and derivatives thereof; vitamin C compounds such as Ascorbic Acid (INCI), Ascorbyl Dipalmitate (INCI), Disodium Ascorbyl Sulfate (INCI) and dipotassium L-ascorbyl diphosphate; vitamin D compounds such as Ergocalciferol (INCI) and Cholecalciferol (INCI); vitamin E compounds such as Tocopherol (INCI), Tocopheryl Acetate (INCI), Tocopheryl Nicotinate (INCI) and Tocopheryl Succinate (INCI); vitamin P compounds such as Biotin (INCI) and Rutin (INCI); nicotinic acids such as Niacin (INCI), Benzyl Nicotinate (INCI) and Niacinamide (INCI); and pantothenic acids such as Calcium Pantothenate (INCI), Panthenol (INCI), pantothenyl ethyl ether and acetyl pantothenyl ethyl ether.

Examples of amino acids include one, two or more selected from Glycine (INCI), Valine (INCI). Leucine (INCI), Isoleucine (INCI), Serine (INCI), Threonine (INCI), Phenylalanine (INCI), Arginine (INCI), Lysine (INCI), Aspartic Acid (INCI), Glutamic Acid (INCI), Cystine (INCI), Cysteine (INCI), Methionine (INCI) and Tryptophan (INCI). An example of a nucleic acid is DNA (INCI). Examples of hormones include Estradiol (INCI) and Ethynyl Estradiol (INCI).

Examples of hair-fixing polymeric compounds include various amphoteric, anionic, cationic and nonionic polymer compounds, one, two or more of which may be selected from polyvinylpyrrolidone-based polymeric compounds such as PVP (INCI) and (VP/VA) Copolymer (INCI); acidic vinyl ether-based polymeric compounds such as (methyl vinyl ether/maleic acid) copolymer (INCI: PVM/MA Copolymer); acidic vinyl polyacetate-type polymeric compounds such as VA/Crotonates Copolymer (INCI), acidic acrylic polymeric compounds such as (meth)acrylic acid/alkyl (meth)acrylate copolymers and (meth)acrylic acid/alkyl (meth)acrylate/alkyl acrylamide copolymers; and amphoteric acrylic polymer compounds such as N-methacryloylethyl-N,N-dimethylammonium/α-N-methylcarboxybetaine/alkyl (meth)acrylate copolymers and (octyl acrylamide/hydroxypropyl acrylate/butylaminoethyl methacrylate) copolymer. Naturally occurring polymeric compounds such as cellulose and derivatives thereof, gelatin and collagen or derivatives thereof may also be suitably used. In addition to the above, resins, fragrances, pH adjustors, chelating agents, algefacients, anti-inflammatory agents, nucleic acids, hormones and inclusion compounds may be included as other ingredients.

The inventive cosmetic is exemplified by skin care cosmetics such as milky lotions, creams, cleansing packs, massages, beauty essences, beauty oils, cleansing agents, deodorants, hand creams, lip creams and wrinkle concealers; makeup cosmetics such as makeup bases, concealers, face powders, liquid foundations, oil-based foundations, rouges, eye shadows, mascaras, eye liners, eyebrows and lipsticks; hair cosmetics such as shampoos, rinses, treatments and setting agents; and ultraviolet-protecting cosmetics such as antiperspirants, sunscreen oils, sunscreen lotions and sunscreen creams.

The shape of these cosmetics may be selected from among various shapes such as liquids, emulsions, creams, solids, pastes, gels, powders, compacts, multilayer lotions, mousses, sprays and sticks.

In addition, the form in which these cosmetics are used may be selected from among various forms, such as aqueous, oil-based, oil-in-water emulsions, water-in-oil emulsion, nonaqueous emulsions, and W/O/W and O/W/O multi-emulsions.

### EXAMPLES

The invention is illustrated more fully below by way of Examples and Comparative Examples, although the invention is not limited by these Examples. In the following Examples, unless noted otherwise, references to "%" in the composition signify percent by weight and references to "ratio" signify a weight ratio.

### [Example 1]

Isopropyl alcohol (23.3 g) was placed in a separable flask and heated at 100°C. Cetyl methacrylate (16.6 g), 2-hydroxyethyl methacrylate (16.6 g), Crosslinking Agent (1) of the formula below (0.34 g), isopropyl alcohol (16.6 g) and Perbutyl O (from NOF Corporation; 2.0 g) were mixed together and then added dropwise to the separable flask over a period of one hour using a dropping funnel. Following dropwise addition, the flask contents were stirred for two hours, after which more Perbutyl O (0.3 g) was added. The system was additionally stirred for three hours, bringing the reaction to completion. The solvent was subsequently driven off under reduced pressure (110°C, ≤1 kPa), giving the target copolymer. The number-average molecular weight Mn was 30,218.

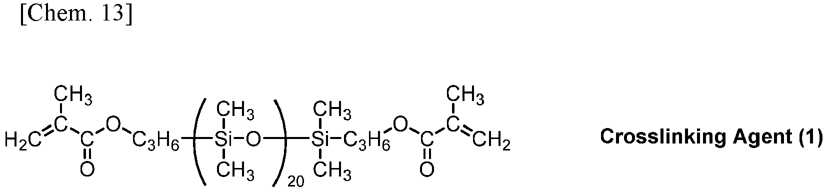

### [Example 2]

Isopropyl alcohol (23.3 g) was placed in a separable flask and heated at 100°C. Cetyl methacrylate (16.6 g), 2-hydroxyethyl methacrylate (16.6 g), Crosslinking Agent (2) of the formula below (0.34 g), isopropyl alcohol (16.6 g) and Perbutyl O (from NOF Corporation; 2.0 g) were mixed together and then added dropwise to the separable flask over a period of one hour using a dropping funnel. Following dropwise addition, the flask contents were stirred for two hours, after which more Perbutyl O (0.3 g) was added. The system was additionally stirred for three hours, bringing the reaction to completion. The solvent was subsequently driven off under reduced pressure (110°C, ≤1 kPa), giving the target copolymer. The number-average molecular weight Mn was 27,164.

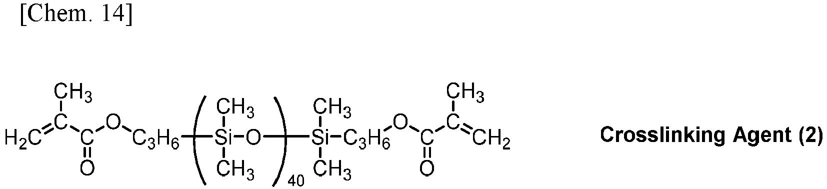

### [Example 3]

Isopropyl alcohol (23.3 g) was placed in a separable flask and heated at 100°C. Cetyl methacrylate (16.6 g), 2-hydroxyethyl methacrylate (16.6 g), Crosslinking Agent (3) of the formula below (0.34 g), isopropyl alcohol (16.6 g) and Perbutyl O (from NOF Corporation; 2.0 g) were mixed together and then added dropwise to the separable flask over a period of one hour using a dropping funnel. Following dropwise addition, the flask contents were stirred for two hours, after which more Perbutyl O (0.3 g) was added. The system was additionally stirred for three hours, bringing the reaction to completion. The solvent was subsequently driven off under reduced pressure (110°C, ≤1 kPa), giving the target copolymer. The number-average molecular weight Mn was 30,133.

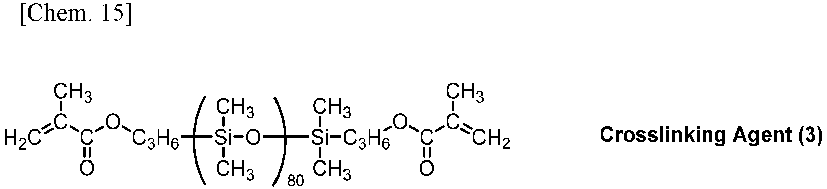

### [Example 4]

Isopropyl alcohol (23.3 g) was placed in a separable flask and heated at 100°C. Behenyl methacrylate (25.0 g), 2-hydroxyethyl methacrylate (8.3 g), Crosslinking Agent (1) of the above-indicated formula (0.34 g), isopropyl alcohol (16.6 g) and Perbutyl O (from NOF Corporation; 2.0 g) were mixed together and then added dropwise to the separable flask over a period of one hour using a dropping funnel. Following dropwise addition, the flask contents were stirred for two hours, after which more Perbutyl O (0.3 g) was added. The system was additionally stirred for three hours, bringing the reaction to completion. The solvent was subsequently driven off under reduced pressure (110°C, ≤1 kPa), giving the target copolymer. The number-average molecular weight Mn was 30,268.

### [Example 5]

Isopropyl alcohol (23.3 g) was placed in a separable flask and heated at 100°C. Behenyl methacrylate (25.0 g), 2-hydroxyethyl methacrylate (8.3 g), Crosslinking Agent (2) of the above-indicated formula (0.34 g), isopropyl alcohol (16.6 g) and Perbutyl O (from NOF Corporation; 2.0 g) were mixed together and then added dropwise to the separable flask over a period of one hour using a dropping funnel. Following dropwise addition, the flask contents were stirred for two hours, after which more Perbutyl O (0.3 g) was added. The system was additionally stirred for three hours, bringing the reaction to completion. The solvent was subsequently driven off under reduced pressure (110°C, ≤1 kPa), giving the target copolymer. The number-average molecular weight Mn was 34,299.

### [Example 6]

Isopropyl alcohol (23.3 g) was placed in a separable flask and heated at 100°C. Behenyl methacrylate (25.0 g), 2-hydroxyethyl methacrylate (8.3 g), Crosslinking Agent (3) of the above-indicated formula (0.34 g), isopropyl alcohol (16.6 g) and Perbutyl O (from NOF Corporation; 2.0 g) were mixed together and then added dropwise to the separable flask over a period of one hour using a dropping funnel. Following dropwise addition, the flask contents were stirred for two hours, after which more Perbutyl O (0.3 g) was added. The system was additionally stirred for three hours, bringing the reaction to completion. The solvent was subsequently driven off under reduced pressure (110°C, ≤1 kPa), giving the target copolymer. The number-average molecular weight Mn was 31,244.

### [Example 7]

Isopropyl alcohol (23.3 g) was placed in a separable flask and heated at 100°C. Behenyl methacrylate (11.4 g), 2-hydroxyethyl methacrylate (8.3 g), Silicone Macromer (1) of the formula shown below (13.6 g), above Crosslinking Agent (2) (0.34 g), isopropyl alcohol (16.6 g) and Perbutyl O (from NOF Corporation; 2.0 g) were mixed together and then added dropwise to the separable flask over a period of one hour using a dropping funnel. Following dropwise addition, the flask contents were stirred for two hours, after which more Perbutyl O (0.3 g) was added. The system was additionally stirred for three hours, bringing the reaction to completion. The solvent was subsequently driven off under reduced pressure (110°C, ≤1 kPa), giving the target copolymer. The number-average molecular weight Mn was 35,125.

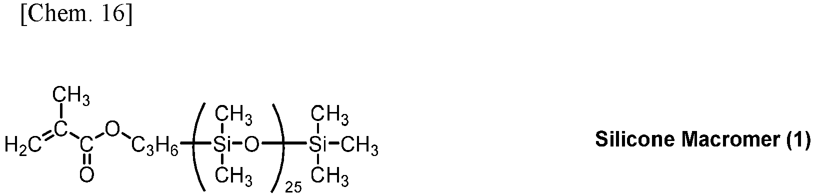

### [Comparative Example 1]

Isopropyl alcohol (23.3 g) was placed in a separable flask and heated at 100°C. Cetyl methacrylate (16.6 g), 2-hydroxyethyl methacrylate (16.6 g), isopropyl alcohol (16.6 g) and Perbutyl O (from NOF Corporation; 2.0 g) were mixed together and then added dropwise to the separable flask over a period of one hour using a dropping funnel. Following dropwise addition, the flask contents were stirred for two hours, after which more Perbutyl O (0.3 g) was added. The system was additionally stirred for three hours, bringing the reaction to completion. The solvent was subsequently driven off under reduced pressure (110°C, ≤1 kPa), giving the target copolymer. The number-average molecular weight Mn was 23,773.

### [Example 2]

Isopropyl alcohol (23.3 g) was placed in a separable flask and heated at 100°C. Cetyl methacrylate (15.0 g), 2-hydroxyethyl methacrylate (18.3 g), Crosslinking Agent (1) of the above-indicated formula (0.34 g), isopropyl alcohol (16.6 g) and Perbutyl O (from NOF Corporation; 2.0 g) were mixed together and then added dropwise to the separable flask over a period of one hour using a dropping funnel. Following dropwise addition, the flask contents were stirred for two hours, after which more Perbutyl O (0.3 g) was added. The system was additionally stirred for three hours, bringing the reaction to completion. The solvent was subsequently driven off under reduced pressure (110°C, ≤1 kPa), giving the target copolymer. The number-average molecular weight Mn was 29,560.

### [Example 3]

Isopropyl alcohol (23.3 g) was placed in a separable flask and heated at 100°C. Cetyl methacrylate (28.3 g), 2-hydroxyethyl methacrylate (5.0 g), Crosslinking Agent (1) of the above-indicated formula (0.34 g), isopropyl alcohol (16.6 g) and Perbutyl O (from NOF Corporation; 2.0 g) were mixed together and then added dropwise to the separable flask over a period of one hour using a dropping funnel. Following dropwise addition, the flask contents were stirred for two hours, after which more Perbutyl O (0.3 g) was added. The system was additionally stirred for three hours, bringing the reaction to completion. The solvent was subsequently driven off under reduced pressure (110°C, ≤1 kPa), giving the target copolymer. The number-average molecular weight Mn was 30,454.

### [Example 4]

Isopropyl alcohol (23.3 g) was placed in a separable flask and heated at 100°C. Cetyl methacrylate (33.3 g), Crosslinking Agent (1) of the above-indicated formula (0.34 g), isopropyl alcohol (16.6 g) and Perbutyl O (from NOF Corporation; 2.0 g) were mixed together and then added dropwise to the separable flask over a period of one hour using a dropping funnel. Following dropwise addition, the flask contents were stirred for two hours, after which more Perbutyl O (0.3 g) was added. The system was additionally stirred for three hours, bringing the reaction to completion. The solvent was subsequently driven off under reduced pressure (110°C, ≤1 kPa), giving the target copolymer. The number-average molecular weight Mn was 27,543.

### [Example 5]

Isopropyl alcohol (23.3 g) was placed in a separable flask and heated at 100°C. 2-Hydroxyethyl methacrylate (33.3 g), isopropyl alcohol (16.6 g), Crosslinking Agent (1) of the above-indicated formula (0.34 g) and Perbutyl O (from NOF Corporation; 2.0 g) were mixed together and then added dropwise to the separable flask over a period of one hour using a dropping funnel. Following dropwise addition, the flask contents were stirred for two hours, after which more Perbutyl O (0.3 g) was added. The system was additionally stirred for three hours, bringing the reaction to completion. The solvent was subsequently driven off under reduced pressure (110°C, ≤1 kPa), giving the target copolymer. The number-average molecular weight Mn was 31,036.

### [Example 6]

Isopropyl alcohol (23.3 g) was placed in a separable flask and heated at 100°C. Behenyl methacrylate (25.0 g), 2-hydroxyethyl methacrylate (8.3 g), isopropyl alcohol (16.6 g) and Perbutyl O (from NOF Corporation; 2.0 g) were mixed together and then added dropwise to the separable flask over a period of one hour using a dropping funnel. Following dropwise addition, the flask contents were stirred for two hours, after which more Perbutyl O (0.3 g) was added. The system was additionally stirred for three hours, bringing the reaction to completion. The solvent was subsequently driven off under reduced pressure (110°C, ≤1 kPa), giving the target copolymer. The number-average molecular weight Mn was 20,786.

### [Example 7]

Isopropyl alcohol (23.3 g) was placed in a separable flask and heated at 100°C. Behenyl methacrylate (11.4 g), 2-hydroxyethyl methacrylate (8.3 g), Silicone Macromer (1) of the above-indicated formula (13.6 g), isopropyl alcohol (16.6 g) and Perbutyl O (from NOF Corporation; 2.0 g) were mixed together and then added dropwise to the separable flask over a period of one hour using a dropping funnel. Following dropwise addition, the flask contents were stirred for two hours, after which more Perbutyl O (0.3 g) was added. The system was additionally stirred for three hours, bringing the reaction to completion. The solvent was subsequently driven off under reduced pressure (110°C, ≤1 kPa), giving the target copolymer. The number-average molecular weight Mn was 33,802.

### [Evaluation of Gelling Ability]

Two grams of the copolymer obtained in, respectively, Examples 1 to 7 and Comparative Examples 1 to 7 was dissolved in 8 g of various oils (Isododecane (INCI), Hydrogenated Polyisobutene (INCI), decamethylcyclopentasiloxane (D5) (INCI: Cyclopentasiloxane)). In each case, the solution was cooled in a 5°C refrigerator, thereby forming a gel, after which it was returned to room temperature (25°C) and the gelling ability was evaluated. The evaluation results were rated as "○" when gelling occurred, "×" when dissolution without gelling occurred, and "insoluble" when the oil did not dissolve. Each of the gels obtained was then placed in a 50°C thermostatic chamber and their temperature stabilities were compared. The evaluation results are shown in Tables 1 and 2. Each of the copolymers of the invention was able to gel the respective oils. On comparing the temperature stabilities, the gels in the Examples of the invention had better temperature stabilities than the gels in the Comparative Examples.

**[Table 1]**

| Composition (g) | | | Example 1 | Example 2 | Example 3 | Example 4 | Example 5 | Example 6 | Example 7 |
|---|---|---|---|---|---|---|---|---|---|
| (a) | 2-Hydroxyethyl methacrylate | | 16.6 | 16.6 | 16.6 | 8.3 | 8.3 | 8.3 | 8.3 |
| (b) | Cetyl methacrylate | | 16.6 | 16.6 | 16.6 | | | | |
| | Behenyl methacrylate | | | | | 25.0 | 25.0 | 25.0 | 11.4 |
| (c) | Crosslinking Agent (1) | | 0.34 | | | 0.34 | | | |
| | Crosslinking Agent (2) | | | 0.34 | | | 0.34 | | 0.34 |
| | Crosslinking Agent (3) | | | | 0.34 | | | 0.34 | |
| (d) | Silicone Macromer (1) | | | | | | | | 13.6 |
| Total | | | 33.54 | 33.54 | 33.54 | 33.64 | 33.64 | 33.64 | 33.64 |
| Number-average molecular weight Mn | | | 30,218 | 27,164 | 30,133 | 30,268 | 34,299 | 31,244 | 35,125 |
| (a) + (b) (%) or (a) + (b) + (c) (%) in overall monomer | | | 99.0 | 99.0 | 99.0 | 99.0 | 99.0 | 99.0 | 99.0 |
| (b) / (a) | | | 1.0 | 1.0 | 1.0 | 3.0 | 3.0 | 3.0 | 1.4 |
| (c) (%) | | | 1.0 | 1.0 | 1.0 | 1.0 | 1.0 | 1.0 | 1.0 |
| Gelling ability | | Oil | isododecane | isododecane | isododecane | hydrogenated polyisobutene | hydrogenated polyisobutene | hydrogenated polyisobutene | D5 |
| | | Result | ○ | ○ | ○ | ○ | ○ | ○ | ○ |
| Temperature stability (50°C) | | | gelled for ≥2 hrs | gelled for ≥2 hrs | gelled for ≥2 hrs | gelled for ≥20 min | gelled for ≥20 min | gelled for ≥20 min | gelled for ≥20 min |

It was also confirmed that results similar to those for the above Examples can be obtained when (b)/(a) is in the range of 0.8 to 4.0 and when the amount of monomer (c) in the overall monomer is in the range of 0.05 to 5 wt%.

**[Table 2]**

| Composition (g) | | | Comparative Example 1 | Comparative Example 2 | Comparative Example 3 | Comparative Example 4 | Comparative Example 5 | Comparative Example 6 | Comparative Example 7 |
|---|---|---|---|---|---|---|---|---|---|
| (a) | 2-Hydroxyethyl methacrylate | | 16.6 | 18.3 | 5.0 | | 33.3 | 8.3 | 8.3 |
| (b) | Cetyl methacrylate | | 16.6 | 15.0 | 28.3 | 33.3 | | | |
| | Behenyl methacrylate | | | | | | | 25.0 | 11.4 |
| (c) | Crosslinking Agent (1) | | | 0.34 | 0.34 | 0.34 | 0.34 | | |
| (d) | Silicone Macromer (1) | | | | | | | | 13.6 |
| Total | | | 33.20 | 33.64 | 33.64 | 33.64 | 33.64 | 33.30 | 33.30 |
| Number-average molecular weight Mn | | | 23,773 | 29,560 | 30,454 | 27,543 | 31,036 | 20,786 | 33,802 |
| (a) + (b) (%) or (a) + (b) + (c) (%) in overall monomer | | | 100.0 | 99.0 | 99.0 | 99.0 | 99.0 | 100.0 | 100.0 |
| (b) / (a) | | | 1.0 | 0.8 | 5.7 | 0.0 | 0.0 | 3.0 | 1.4 |
| (c) (%) | | | 0 | 1.0 | 1.0 | 1.0 | 1.0 | 0 | 0 |
| Gelling ability | | Oil | isododecane | isododecane | isododecane | isododecane | isododecane | hydrogenated polyisobutene | D5 |
| | | Result | ○ | insoluble | × | × | insoluble | ○ | ○ |
| Temperature stability (50°C) | | | dissolved in 2 hrs | - | - | - | - | dissolved in 10 min | dissolved in 10 min |

Formulation Examples of oil-based cosmetics and oil-in-water emulsion type cosmetics formulated with copolymers of the invention are given below. The invention is not limited by these Formulation Examples. The amounts given below indicate the amounts in which the compounded ingredients are included.

### [Formulation Example 1] Lipstick

| | (Ingredients) | | Weight (%) |
|---|---|---|---|
| 1. Polyethylene (INCI) | | | 14.0 |
| 2. Microcrystalline Wax (INCI) | | | 4.0 |
| 3. Polybutene (INCI) | | | 10.0 |
| 4. Oil gelling agent mentioned in Example 5 | | | 15.0 |
| 5. Cetyl octanoate (INCI: Cetyl Ethylhexanoate) | | | 20.0 |
| 6. Triisostearin (INCI) | | | 37.0 |
| 7. Pigment | | | q.s. |
| 8. Preservative | | | q.s. |
| 9. Fragrance | | | q.s. |
| | | Total | 100.0 |

### (Method of Preparation)

A: Ingredients 5 and 6 were uniformly mixed.
B: Ingredients 1 to 4 were heated (90°C) and dissolved, then added to A and rendered uniform.
C: Ingredients 7 to 9 were added to B at 80°C and rendered uniform.

The lipstick obtained in this way went on lightly without being oily or powdery, giving the lips a fresh feeling. Moreover, it had a good water resistance and water repellency, was long-lasting, and also had excellent stability.

### [Formulation Example 2] Oil-in-Water Cream

| | (Ingredients) | | Weight (%) |
|---|---|---|---|
| 1. | Oil gelling agent mentioned in Example 2 | | 8.0 |
| 2. | Crosslinked methyl phenyl polysiloxane (*1) ((dimethicone/phenyl vinyl dimethicone) crosspolymer (INCI: Diphenylsiloxy Phenyl Trimethicone)) | | 2.0 |
| 3. | Isotridecyl Isononanoate (INCI) | | 5.0 |
| 4. | DPG (INCI) | | 7.0 |
| 5. | Glycerin (INCI) | | 5.0 |
| 6. | Methylcellulose (INCI) (2% aqueous solution) (*2) | | 7.0 |
| 7. | Polyacrylamide-based emulsifying agent (*3) (Polyacrylamide (INCI), (C13-14) Isoparaffin (INCI), Laureth-7 (INCI), water) | | 2.0 |
| 8. | Guanine (INCI) | | 1.0 |
| 9. | Preservative | | q.s. |
| 10. | Fragrance | | q.s. |
| 11. | Purified water | | 63.0 |
| | | Total | 100.0 |

| | | | |
|---|---|---|---|
| (* 1) KSG-18, from Shin-Etsu Chemical Co., Ltd. (*2) Metolose SM-4000, from Shin-Etsu Chemical Co., Ltd. (*3) Sepigel 305, from SEPPIC | | | |

### (Method of Preparation)

A: Ingredients 4 to 11 were mixed together.
B: Ingredients 1 to 3 were mixed together, A was added thereto, and the mixture was emulsified by stirring.

The cream obtained in this way had a fine texture and spread lightly without being sticky or oily, and moreover was moist and dewy, leaving the skin feeling fresh. This O/W cream was also confirmed to be very long-lasting and to have excellent stability, remaining unchanged with fluctuations in temperature and over time.

### [Formulation Example 3] W/O Cream Foundation

| | (Ingredients) | | Weight (%) |
|---|---|---|---|
| 1. Oil gelling agent mentioned in Example 2 | | | 4.0 |
| 2. Polyether/Alkyl Co-Modified Branched Silicone (*1) | | | 1.0 |
| | (INCI: PEG-9/Lauryl Polydimethylsiloxyethyl Dimethicone Crosspolymer) | | |
| 3. Triethylhexanoin (INCI) | | | 2.0 |
| 4. Cetyl octanoate (INCI: Cetyl Ethylhexanoate) | | | 5.0 |
| 5. Isotridecyl Isononanoate (INCI) | | | 9.0 |
| 6. Hybrid silicone composite powder (*2) | | | 3.0 |
| | (INCI: (Vinyl Dimethicone/Methicone Silsesquioxane) Crosspolymer) | | |
| 7. Polyglycerol co-modified branched silicone (*3) | | | 0.6 |
| | (INCI: Polyglyceryl-3-Disiloxane Dimethicone) | | |
| 8. Polyglycerol co-modified branched silicone (*4) | | | 0.3 |
| | (INCI: Laurylpolyglyceryl-3 Polydimethylsiloxyethyl Dimethicone) | | |
| 9. Alkyl/silicone treated pigment (*5) | | | 10.0 |
| | (INCI: Triethoxysilylethyl Polydimethylsiloxyethylhexyl Dimethicone) | | |
| 10. BG (INCI: Butylene Glycol) | | | 5.0 |
| 11. Sodium chloride | | | 0.5 |
| 12. Sodium citrate | | | 0.2 |
| 13. Preservative | | | q.s. |
| 14. Fragrance | | | q.s. |
| 15. Purified water | | | 59.4 |
| | | Total | 100.0 |

| | | | |
|---|---|---|---|
| (*1) KF-6038, from Shin-Etsu Chemical Co., Ltd. (*2) KSP-100, from Shin-Etsu Chemical Co., Ltd. (*3) KF-6100, from Shin-Etsu Chemical Co., Ltd. (*4) KF-6105, from Shin-Etsu Chemical Co., Ltd. (*5) KF-9909, from Shin-Etsu Chemical Co., Ltd. | | | |

### (Method of Preparation)

A: Ingredients 1 to 6 were uniformly mixed.
B: Ingredients 7 to 10 were uniformly mixed.
C: Ingredients 11 to 13 and some of 15 were mixed together and dissolved.
D: B was added to the balance of Ingredient 15 and rendered uniform.
E: C was added to A and emulsified
F: D was added to E and emulsified. Last of all, Ingredient 14 was added and rendered uniform.

The W/O cream foundation obtained in this way was not sticky and spread easily. In addition, it had good pigment dispersibility, adhered well to the skin and felt natural, forming a very clean film with a matte finish.

### [Formulation Example 4] Eye Color

| | (Ingredients) | | Weight (%) |
|---|---|---|---|
| 1. Glycol Distearate (INCI) | | | 12.0 |
| 2. Oil gelling agent mentioned in Example 5 | | | 5.0 |
| 3. Isotridecyl Isononanoate (INCI) | | | 35.0 |
| 4. Candelilla wax (INCI: Euphorbia Cerifera (Candelilla) Wax) | | | 2.0 |
| 5. Lecithin (INCI) | | | 0.2 |
| 6. Hybrid silicone composite powder (*1) | | | 3.0 |
| | (INCI: (Vinyl Dimethicone/Methicone Silsesquioxane) Crosspolymer) | | |
| 7. Alkyl/silicone treated pigment (*2) | | | q.s. |
| | (Iron Oxides (INCI), Triethoxysilylethyl Polydimethylsiloxyethylhexyl Dimethicone (INCI)) | | |
| 8. Mica-treated titanium oxide | | | q.s. |
| | | Total | 100.0 |

| | | | |
|---|---|---|---|
| (*1) KSP-100, from Shin-Etsu Chemical Co., Ltd. (*2) KTP-09, from Shin-Etsu Chemical Co., Ltd. | | | |

A: Ingredients 2 and 3 were mixed together.
B: Ingredients 6 to 8 were mixed together
C: Ingredients 1, 4 and 5 were mixed together, A was added thereto and the mixture was warmed.
D: C was added to B, and the mixture was poured into a container.

The eye color obtained in this way was not oily or sticky, spread lightly and had a fresh feeling on use. In addition, it adhered well to the skin, felt natural and was very long-lasting.

Cosmetics formulated with the oil gelling agent of the invention spread lightly and leave the skin feeling smooth and dry, providing the user with a feeling of freshness. Hence, by applying the cosmetic of the invention, a smooth, soft emollient effect is imparted without the loss of a suitable degree of moisture transpiration, enabling a broad range of properties, from a natural luster to a matte feel, to be conferred. The cosmetics of the invention thus have an excellent usability and also a good stability over time.

### [Industrial Applicability]

The cosmetics of the invention have an excellent feeling on use and a good stability, making them highly useful in practice. Also, the composition of the invention, being a starting ingredient for cosmetics in practical use, has a high industrial utility.

The invention is not limited to the embodiments described above, which are presented here for the purpose of illustration. Any embodiments having substantially the same constitution as the technical ideas set forth in the claims and exhibiting similar working effects are encompassed within the technical scope of the invention.

## Claims

1. An oil gelling agent which is a copolymer comprising, as constituent units:
(a) a hydrophilic (meth)acrylic monomer selected from the group consisting of monomers of general formula (1) and monomers of general formula (2) below 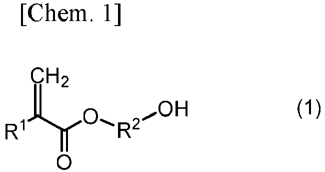 (wherein R¹ is a hydrogen atom or a methyl group, and R² is a linear or branched alkylene group of 2 to 10 carbon atoms) 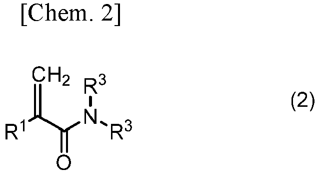 (wherein R¹ is a hydrogen atom or a methyl group; and each R³ is independently a hydrogen atom, a linear or branched alkyl group of 1 to 10 carbon atoms or a hydroxyalkyl group of 1 to 5 carbon atoms),
(b) a hydrophobic (meth)acrylic monomer of general formula (3) below 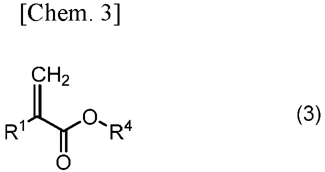 (wherein R¹ is a hydrogen atom or a methyl group, and R⁴ is a linear alkyl group of 1 to 24 carbon atoms), and
(c) a crosslinker monomer having two or more radical polymerizable functional groups per molecule, wherein the weight ratio (b)/(a) between monomers (a) and (b) is from 0.8 to 4.0 and monomer (c) is included in an amount of from 0.05 to 5 wt% of all the monomers.

2. The oil gelling agent of claim 1, wherein the crosslinker monomer (c) is a di(meth)acrylate-terminated silicone of general formula (4) below 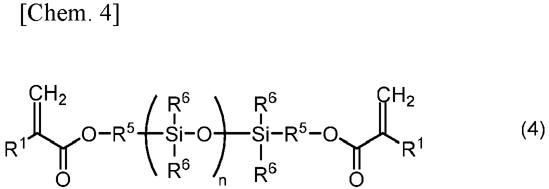 (wherein R¹ is a hydrogen atom or a methyl group; each R⁵ is independently a divalent organic group; each R⁶ is independently a hydrogen atom, a substituted or unsubstituted alkyl group of 1 to 10 carbon atoms, or a substituted or unsubstituted aryl group of 6 to 22 carbon atoms; and n is from 1 to 100).

3. The oil gelling agent of claim 1 or 2, further comprising:
(d) a monomer consisting of a silicone macromer of general formula (5) below 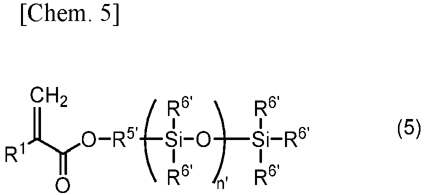 (wherein R¹ is a hydrogen atom or a methyl group; R^{5'} is a divalent organic group; each R^{6'} is independently a hydrogen atom, a substituted or unsubstituted alkyl group of 1 to 10 carbon atoms or a substituted or unsubstituted aryl group of 6 to 22 carbon atoms; and n' is from 1 to 100) as from 10 to 50 wt% of the constituent units in all the monomers.

4. A cosmetic comprising the oil gelling agent of any one of claims 1 to 3.
